# EUROPEAN PATENT APPLICATION

(11) **EP 3 808 759 A1**
(43) Date of publication of application: **21.04.2021**
(21) Application number: 19820266.5
(22) Date of filing: 28.05.2019
(51) Int. Cl.: C07K 1/06, C07K 1/10

(54) **METHOD FOR PRODUCING AMIDE**

(30) Priority: 15.06.2018 JP 2018114782
(71) Applicant: Yokogawa Electric Corporation, Tokyo 180-8750 (JP)
(72) Inventor: FUSE Shinichiro, Tokyo 152-8550 (JP); NAKAMURA Hiroyuki, Tokyo 152-8550 (JP); OTAKE Yuma, Tokyo 152-8550 (JP); OGAWA Jun-ichi, Musashino-shi, Tokyo 180-8750 (JP); MIYAZAKI Shun-ichi, Musashino-shi, Tokyo 180-8750 (JP); ITO Atsushi, Musashino-shi, Tokyo 180-8750 (JP)
(74) Representative: Winter, Brandl - Partnerschaft mbB
(86) International application number: PCT/JP2019/021107
(87) International publication number: WO 2019/239880

(57) **Abstract**

A method for producing an amide includes: reacting arginines, arginine derivatives or arginine analogs in which two amino groups or imino groups in the side chain are protected by protecting groups with a halogenated formate ester and then reacting with an amine.

## Description

### [Technical Field]

The present invention relates to a method for producing an amide.

Priority is claimed on Japanese Patent Application No. 2018-114782, filed June 15, 2018, the content of which is incorporated herein by reference.

### [Background Art]

In peptide synthesis, a carboxylic group of an amino acid is activated and reacted with an amino group of the amino acid, a coupling reaction is caused to form amide bonds, these operations are repeated, and thus the amino acid is sequentially extended. Several methods are known as methods of activating carboxylic groups. There are a method of synthesizing peptides while minimizing isomerization and production of byproducts using a condensing agent having a low degree of activation and a method of synthesizing peptides using an activation agent in a short time.

Examples of a method of activating the carboxylic group using a highly active activation agent include acid chloride methods and acid anhydride methods. Compared with an activation method using a condensing agent having a low degree of activation, these acid chloride methods and acid anhydride methods have advantages such as low unit price, a small amount of produced byproducts derived from an activation agent, and the like because the structure of the activation agent is simpler.

The acid anhydride methods are classified into a symmetric acid anhydride method and a mixed acid anhydride method.

For example, in Non Patent Literature 1 to 2, a method of synthesizing an amide using a symmetric acid anhydride as an active species of a carboxylic acid is disclosed.

The symmetric acid anhydride method disclosed in Non Patent Literature 1 to 2 can be said to be a method including a first step in which a symmetric acid anhydride is produced by a condensation reaction between carboxylic acids and a second step in which a coupling reaction between the symmetric acid anhydride and an amine is performed.

In addition, for example, Non Patent Literature 3 discloses a method of synthesizing an amide using a mixed acid anhydride as an active species of a carboxylic acid.

It is described in Non Patent Literature 3 that a carboxylic acid and isopropyl chloroformate are mixed with a first micro mixer, a mixed acid anhydride is synthesized in a short time, and subsequently, a solution containing the mixed acid anhydride, an amine and a catalyst (base) are immediately mixed with a second micro mixer to perform amidation so that the synthesized mixed acid anhydride is not racemized.

The mixed acid anhydride method disclosed in Non Patent Literature 3 can be said to be a method including a first step in which a carboxylic acid reacts with chloroformate to obtain a mixed acid anhydride, a second step in which a base is added to the mixed acid anhydride to obtain an acylpyridinium species, and a third step in which a coupling reaction between the acylpyridinium species and an amine is performed to obtain an amide.

### [Citation List]

### [Non Patent Literature]

[Non Patent Literature 1]
   "Efficient Amide Bond Formation through a Rapid and StrongActivation of Carboxylic Acids in a Microflow Reactor," Fuse, S. Mifune, Y. Takahashi, T., Angew Chem. Int. Ed. 53, 851-855 (2014).
[Non Patent Literature 2]
   "Total synthesis of feglymycin based on a linear/convergent hybrid approach using micro-flow amide bond formation," Fuse, S. Mifune, Y. Nakamura, H. Tanaka, H. Nat. Commun. 7, 13491 (2016).
[Non Patent Literature 3]
   Yuma Otake, Hiroyuki Nakamura, Shinichiro Fuse, "An efficient synthesis of N-methylated peptide using micro-flow methodology," March 16, 2017, The 97th Annual Meeting of the Chemical Society of Japan, 3F4-14

### [Summary of Invention]

### [Technical Problem]

In a symmetric acid anhydride method, when arginine or an arginine derivative is used as an amine, there is a problem that the reaction hardly proceeds. In addition, even if the reaction proceeds, there is a problem that side reactions such as isomerization of the amino acid and production of δ-lactam occur with a high probability.

In a mixed acid anhydride method, even if arginine or an arginine derivative is used as an amine, the reaction can proceed. However, the problem that side reactions such as isomerization of the amino acid and production of δ-lactam occurs with a high probability is still unsolved.

The present invention has been made in order to address the above problems, and an object of the present invention is to provide a method for producing an amide in which, in the reaction in which carboxylic groups are activated and reacted with an amino group, a coupling reaction is caused to form amide bonds, the reaction efficiency is favorable and side reactions are unlikely to occur.

### [Solution to Problem]

That, is the present invention includes the following aspects.
(1) A method for producing an amide, the method including: reacting arginines, arginine derivatives or arginine analogs in which two amino groups or imino groups in the side chain are protected by protecting groups with a halogenated formate ester and then reacting with an amine.
(2) The method for producing an amide according to the (1), the method including: reacting arginines, arginine derivatives or arginine analogs in which two amino groups or imino groups in the side chain are protected by protecting groups with a halogenated formate ester and then reacting with a base and reacting with an amine.
(3) A method for producing an amide, the method including: mixing a product obtained by reacting a mixture obtained by mixing arginines, arginine derivatives or arginine analogs in which two amino groups or imino groups in the side chain are protected by protecting groups and a halogenated formate ester, and an amine.
(4) The method for producing an amide according to (3), the method including: mixing a product obtained by reacting a mixture obtained by mixing arginines, arginine derivatives or arginine analogs in which two amino groups or imino groups in the side chain are protected by protecting groups and a halogenated formate ester, a base, and an amine.
(5) The method for producing an amide according to the (2) or (4), wherein the base is any one or more selected from the group consisting of pyridine, pyridine derivatives, imidazole, imidazole derivatives and 1,4-diazabicyclo [2,2,2] octane.
(6) The method for producing an amide according to the (2) or (4), wherein the base is any one or more selected from the group consisting of 4-morpholinopyridine, N,N-dimethyl-4-aminopyridine, 4-pyrrolidinopyridine, pyridine, 4-methoxypyridine, imidazole, N-methylimidazole and 1,4-diazabicyclo [2,2,2] octane.
(7) The method for producing an amide according to any one of the (1) to (6), wherein the two protecting groups are carbamate-based protecting groups or sulfonamide-based protecting groups.
(8) The method for producing an amide according to any one of the (1) to (7), wherein the halogenated formate ester is any one or more selected from the group consisting of isopropyl chloroformate, isobutyl chloroformate, isopropyl bromomate and isobutyl bromomate.
(9) The method for producing an amide according to any one of the (1) to (8), wherein the amine is an amino acid or an amino acid derivative.
(10) The method for producing an amide according to any one of the (1) to (9), wherein the nucleophilicity of the amine is lower than the nucleophilicity of 18 amino acids excluding valine and isoleucine from 20 amino acids which constitute proteins and are encoded as genetic information.
(11) The method for producing an amide according to the (9) or (10), wherein the amine is valine, isoleucine an N-alkylated amino acid, or derivatives thereof.
(12) The method for producing an amide according to any one of the (1) to (11), wherein the reaction with the amine is performed in a distribution system reaction device.
(13) The method for producing an amide according to the (12), wherein arginines, arginine derivatives or arginine analogs in which two amino groups or imino groups in the side chain are protected by protecting groups are additionally reacted with a halogenated formate ester in the distribution system reaction device.

### [Advantageous Effects of Invention]

According to the present invention, it is possible to provide a method for producing an amide which has favorable reaction efficiency and is unlikely to cause a side reaction.

### [Brief Description of Drawings]

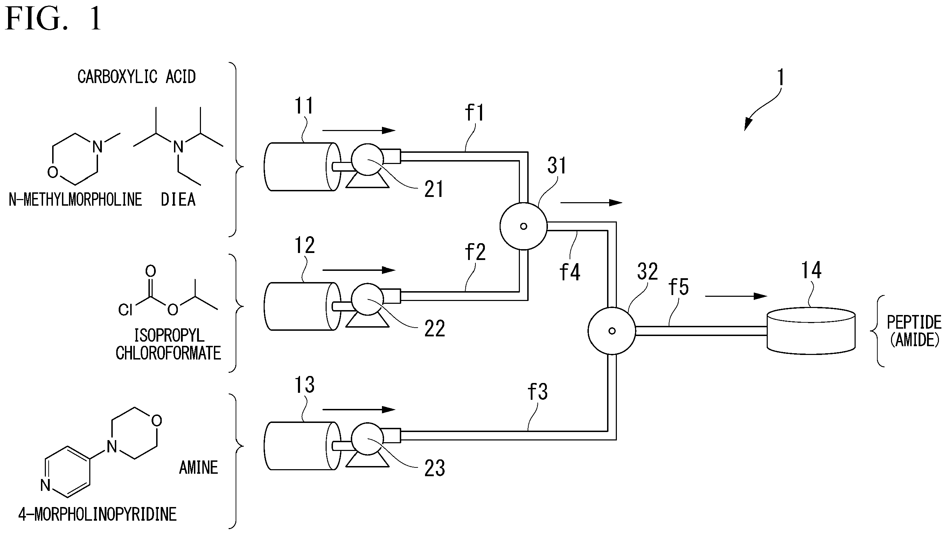

Fig. 1 is a schematic view showing a schematic configuration of a distribution system reaction device 1.

### [Description of Embodiments]

A method for producing an amide according to an embodiment of the present invention will be described below.

### «Method for producing amide»

### [First embodiment]

The method for producing an amide according to the embodiment includes reacting arginines, arginine derivatives or arginine analogs in which two amino groups or imino groups in the side chain are protected by protecting groups (in this specification, hereinafter sometimes referred to as "arginines") with a halogenated formate ester, and then reacting with a base and reacting with an amine.

The method for producing an amide according to the embodiment may be a method including mixing a product obtained by reacting a mixture obtained by mixing arginines and a halogenated formate ester, a base, and an amine. Here, the product obtained by reacting a mixture obtained by mixing arginines and a halogenated formate ester can include a mixed acid anhydride.

Here, the base may be one that produces a cationically active species or a base (excluding the amine).

Here, the term "mixing" as used herein refers to an operation of adding substances such as raw materials to the reaction system, and when these are mixed in the reaction system, raw materials and the like may be changed to substances different from those before addition.

In the method for producing an amide according to the embodiment, arginines in which two amino groups or imino groups in the side chain are protected by protecting groups are used as carboxylic acids in an amide bond formation. The production method may include the following Processes 1 to 3.

Process 1: a process in which arginines in which two amino groups or imino groups in the side chain are protected by a protecting group are reacted with a halogenated formate ester to obtain a mixed acid anhydride.

Process 2: a process in which the mixed acid anhydride obtained in Process 1 is reacted with a base to obtain a cationically active species.

Process 3: a process in which the cationically active species obtained in Process 2 is reacted with an amine to produce an amide.

The above processes will be described below. Here, the reaction of the method for producing an amide according to the present invention is not limited to reactions exemplified in the following processes.

### <Process 1>

Process 1 is a process in which arginines in which two amino groups or imino groups in the side chain are protected by a protecting group are reacted with a halogenated formate ester to obtain a mixed acid anhydride.

The arginines preferably have an α-amino acid framework. In addition, generally, since amino acids constituting peptides or proteins in a living body are of an L-type, the arginines are preferably an L-type. The arginines may be compounds represented by the following General Formula (1). 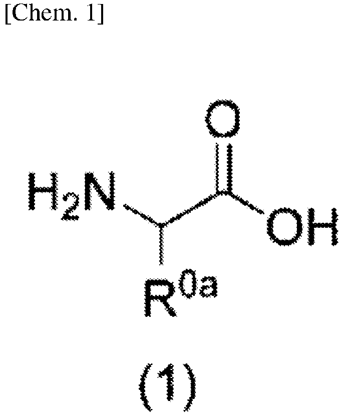 (in the formula, R^{0a} represents a side chain of arginines)

Arginines may be deprotonated into carboxylate ions and may be represented by the following General Formula (1i). 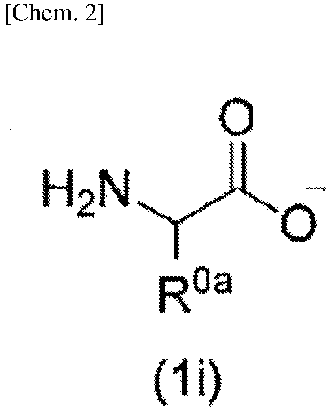 (in the formula, R^{0a} represents a side chain of arginines)

Deprotonation of the arginines can be achieved, for example, by placing the arginines in the presence of a base having low nucleophilicity such as N,N-diisopropylethylamine (DIEA) in the reaction system. The presence of a base means, for example, in a solvent in which a base is added. The type of the base is not particularly limited as long as it allows the arginines to be deprotonated in the reaction system.

R^{0a} in Formula (1-1) is a group represented by the following Formula (R^{0a-}a) when the arginines are arginines.

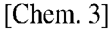

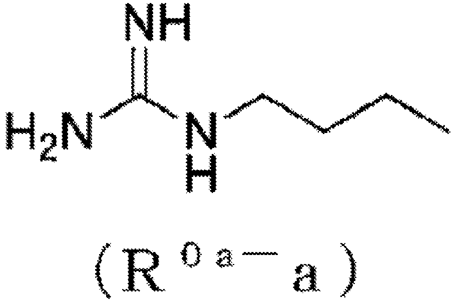

The arginines according to the embodiment are limited to those in which two amino groups or imino groups in the side chain are protected by protecting groups. Here, the fact that the functional group is protected means that atoms constituting the functional group are substituted with a protecting group. Examples of side chains of arginines in which two amino groups or imino groups are protected by protecting groups include a group represented by the following General Formula (R^{0a-}b). 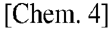 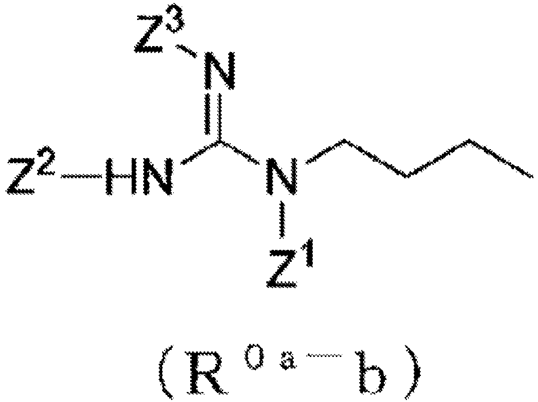 (in the formula, Z¹, Z² and Z³ each independently represent a hydrogen atom or a protecting group, and two or more of Z¹, Z² and Z³ are protecting groups)

When the two amino groups or imino groups are protected, side reactions including isomerization and δ-lactam production are significantly minimized in activation conditions via the acid anhydride.

The protecting group in the group represented by General Formula (R^{0a-}b) is not particularly limited as long as it has a function of inactivating a reactive functional group. Examples of protecting groups in the group represented by General Formula (R^{0a-}b) include those exemplified as the protecting groups to be described below, and include those exemplified as protecting groups in amino groups to be described below, and a carbamate-based protecting group or a sulfonamide-based protecting group is preferable. The two or more protecting groups of Z¹, Z² and Z³ may all be the same or some may be different from each other. In order to minimize the side reaction, more preferably, at least two Z¹ and Z² among the protecting groups Z¹, Z² and Z³ are protected by protecting groups.

Arginine derivatives or arginine analogs in the arginines may be a compound having substantially the same properties as arginine, and may be a natural type that occurs naturally or a type which has modifications such as alternation, addition, or substitution of a functional group different from those of the natural type. The arginine derivatives or arginine analogs preferably have a group represented by General Formula (R^{0a-}b)as a side chain, which may have a substituent. Regarding those having a substituent, those in which one or more hydrogen atoms of the group represented by General Formula (R^{0a-}b) are substituted with other groups may be exemplified.

As an example of a case having substantially the same properties as an arginine, a case in which arginine derivatives or arginine analogs can be incorporated into an enzyme that uses an arginine as a substrate and a case in which arginine derivatives or arginine analogs can be bound to molecules that bind to an arginine may be exemplified.

As an example of arginine derivatives, a protected amino acid in which a functional group is protected by a protecting group may be exemplified. The protecting group has a function of inactivating a reactive functional group. It is possible to deprotect the protecting group and return the protected functional group to its unprotected state. Here, the fact that the functional group is protected means that atoms constituting the functional group are substituted with a protecting group. Examples of sites protected by a protecting group include amino groups and/or carboxylic groups in addition to the side chain exemplified above. In Process 1, it is preferable that amino groups and functional groups in the side chain be protected so that the reaction of the reactive functional group other than carboxylic groups is prevented.

The type of the protecting group is not particularly limited, and can be appropriately selected depending on the type of the functional group to be protected. Examples of amino group protecting groups include carbamate-based, sulfonamide-based, acyl-based, and alkyl-based protecting groups, and the present invention is not limited thereto.

Examples of carbamate-based protecting groups include 2-benzyloxycarbonyl groups (sometimes abbreviated as -Z or -Cbz), tert-butyloxycarbonyl groups (sometimes abbreviated as -Boc), allyloxycarbonyl groups (sometimes abbreviated as -Alloc), 2,2,2-trichloroethoxycarbonyl groups (sometimes abbreviated as -Troc), 2-(trimethylsilyl)ethoxycarbonyl groups (sometimes abbreviated as -Teoc), 9-fluorenylmethyloxycarbonyl groups (sometimes abbreviated as -Fmoc), p-nitrobenzyloxycarbonyl groups (sometimes abbreviated as -Z(NO₂)), and p-biphenylisopropyloxycarbonyl groups (sometimes abbreviated as -Bpoc).

Examples of sulfonamide-based protecting groups include p-toluenesulfonyl groups (sometimes abbreviated as -Ts or -Tos), 2-nitrobenzene sulfonyl groups (sometimes abbreviated as -Ns), 2,2,4,6,7-pentamethyldihydrobenzofuran-5-sulfonyl (sometimes abbreviated as -Pbf), 2,2,5,7,8-pentamethylchroman-6-sulfonyl (sometimes abbreviated as -Pmc), and 1,2-dimethylindole-3-sulfonyl (sometimes abbreviated as - MIS).

In Process 1 in the method for producing an amide according to the embodiment, arginines represented by the following General Formula (1) are reacted with a halogenated formate ester represented by the following General Formula (1)' to obtain a mixed acid anhydride represented by the following General Formula (2). 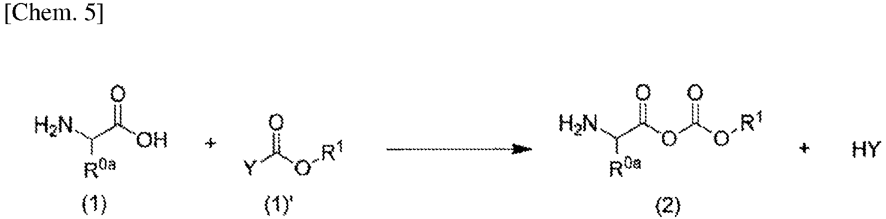 (in the formula, R^{0a} represents a side chain of arginines, R¹ represents a hydrogen atom or a hydrocarbon group, and Y represents a halogen atom)

The hydrocarbon group for R¹ may be an aliphatic hydrocarbon group or an aromatic hydrocarbon group (aryl group). The aliphatic hydrocarbon group may be a saturated aliphatic hydrocarbon group (alkyl group) or an unsaturated aliphatic hydrocarbon group and is preferably an alkyl group.

The aliphatic hydrocarbon group may have 1 to 20 carbon atoms or 1 to 15 carbon atoms.

The alkyl group may be linear, branched or cyclic. The cyclic alkyl group may be either monocyclic or polycyclic. The alkyl group may have 1 to 20 carbon atoms, 1 to 10 carbon atoms, or 1 to 5 carbon atoms.

Examples of linear or branched alkyl groups include methyl groups, ethyl groups, n-propyl groups, isopropyl groups, n-butyl groups, isobutyl groups, sec-butyl groups, tert-butyl groups, n-pentyl groups, isopentyl groups, neopentyl groups, tert-pentyl groups, 1-methylbutyl groups, n-hexyl groups, 2-methylpentyl groups, 3-methylpentyl groups, 2,2-dimethylbutyl groups, 2,3-dimethylbutyl groups, n-heptyl groups, 2-methylhexyl groups, 3-methylhexyl groups, 2,2-dimethylpentyl groups, 2,3-dimethylpentyl groups, 2,4-dimethylpentyl groups, 3,3-dimethylpentyl groups, 3-ethylpentyl groups, 2,2,3-trimethylbutyl groups, n-octyl groups, isooctyl groups, nonyl groups, decyl groups, undecylic groups, dodecyl groups, tridecylic groups, tetradecyl groups, pentadecyl groups, hexadecyl groups, heptadecyl groups, octadecyl groups, nonadecyl groups, and icosyl groups.

The halogen atom for Y is an element belonging to Group 17 in the periodic table such as F, Cl, Br, and I, and is preferably Cl or Br.

In order to more effectively minimize the side reactions, in the halogenated formate ester represented by General Formula (1)', the halogen atom for Y is Cl or Br, and the hydrocarbon group for R¹ is preferably a branched alkyl group having 1 to 5 carbon atoms, and is more preferably any one or more selected from the group consisting of isopropyl chloroformate, isobutyl chloroformate, isopropyl bromomate and isobutyl bromomate.

Here, in the reaction in Process 1, a halogenated formate ester and a reagent (base) such as N-methylmorpholine that activates the halogenated formate ester are used together, and these are reacted, the halogenated formate ester is activated, and the reaction can proceed easily. Here, the activated halogenated formate ester is also included in the concept of halogenated formate ester. Examples of reagents that activate the halogenated formate ester include tertiary amines, 4-methylmorpholine, pyridine, pyridine derivatives, imidazole, imidazole derivatives and 1,4-diazabicyclo [2,2,2] octane. Examples of pyridine derivatives and imidazole derivatives include those exemplified in Process 2 to be described below. Regarding the tertiary amine, an amine in which at least one of groups bonded to N atoms of an amine is a methyl group is preferable. More preferably, two groups bonded to N atoms of an amine are methyl groups. When at least one of groups bonded to N atoms of the tertiary amine is a methyl group, the steric hindrance around the N atoms can be reduced and the reaction efficiency of the halogenated formate ester can be improved.

### <Process 2>

Process 2 is a process in which the mixed acid anhydride obtained in Process 1 is reacted with a base to obtain a cationically active species.

In Process 2 in the method for producing an amide according to the embodiment, a mixed acid anhydride represented by the following General Formula (2) is reacted with a base represented by B to obtain a cationically active species represented by the following General Formula (4). Here, in the reaction, a compound represented by the following General Formula (5) is produced as a counter anion of a cationically active species. 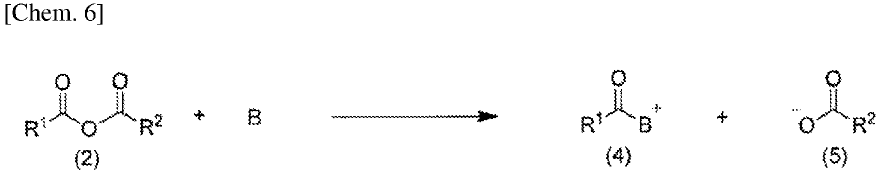 (in the formula (4) and the formula (5), R^{0a} and R¹ mean the same those of the R^{0a} and R¹ in the formula (2))

The base in Process 2 reacts with the acid anhydride to produce a cationically active species, and is preferably a base having high nucleophilicity and more preferably any one or more selected from the group consisting of pyridine, pyridine derivatives, imidazole, imidazole derivatives and 1,4-diazabicyclo [2,2,2] octane.

The pyridine derivative may be one in which one or more hydrogen atoms of pyridine are substituted with other groups and is not particularly limited as long as it has properties of a base, and the pyridine and pyridine derivative are preferably a compound represented by the following General Formula (3-1). 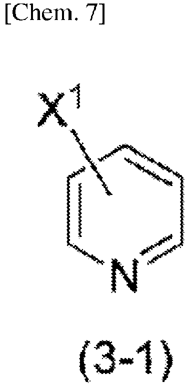 (in the formula, X¹ represents a hydrogen atom or any group selected from among the groups represented by the following Formulae (a) to (c)) 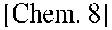 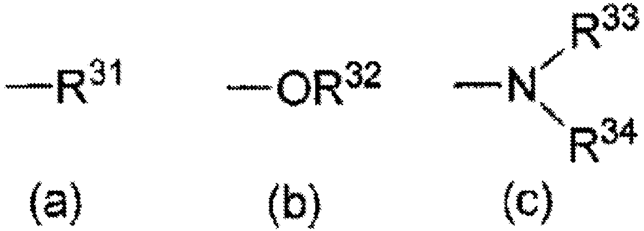 (in the formula, R³¹, R³², R³³ and R³⁴ each independently represent an alkyl group; R³³ and R³⁴ may be bonded to each other to form a ring, and one methylene group that is not directly bonded to R³³ or R³⁴ in the alkyl group may be substituted with an oxygen atom)

The alkyl group for R³¹, R³², R³³ and R³⁴ may be linear, branched or cyclic. The cyclic alkyl group may be either monocyclic or polycyclic. The alkyl group may have 1 to 20 carbon atoms, 1 to 15 carbon atoms, or 1 to 10 carbon atoms.

The linear or branched alkyl groups are the above-described R₁, for example.

The compound represented by General Formula (3-1) is preferably a compound represented by the following General Formula (3-1-1). When X¹ is any group selected from among the groups represented by Formulae (a) to (c) other than a hydrogen atom, X¹ effectively functions as an electron donating group according to bonding to a relevant position, and the nucleophilicity of N atoms of a pyridine ring tends to become better. 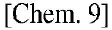 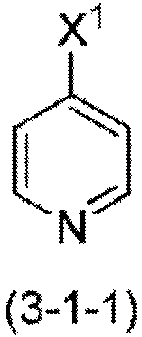 (in Formula (3-1-1), X¹ has the same meaning as X¹ in Formula (3-1))

In the compound represented by General Formula (3-1), X¹ is a group represented by Formula (c), R³³ and R³⁴ are bonded to each other to form a ring, and regarding a case in which one methylene group that is not directly bonded to R³³ or R³⁴ in the alkyl group is substituted with an oxygen atom, 4-morpholinopyridine represented by the following Formula (3-1-2) is included.

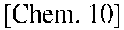

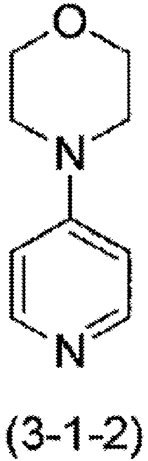

Preferable examples of pyridine and pyridine derivatives include pyridine, the above 4-morpholinopyridine, N,N-dimethyl-4-aminopyridine, 4-pyrrolidinopyridine and 4-methoxypyridine. Among these, 4-morpholinopyridine and N,N-dimethyl-4-aminopyridine are particularly preferably used because an amide synthesis yield per unit time is high and it is possible to significantly reduce the amount of side-reaction products produced.

When the above exemplified pyridine and pyridine derivative are used, the cationically active species is an acylpyridinium cation (an acylpyridinium species). The acylpyridinium species has high electrophilicity. Therefore, even the reaction with an amine having low nucleophilicity to be described below can proceed at a very high rate, and it is possible to significantly reduce the amount of side-reaction products produced.

The imidazole derivative may be one in which one or more hydrogen atoms of imidazole are substituted with other groups and is not particularly limited as long as it has properties of a base, but the imidazole and imidazole derivative are preferably a compound represented by the following General Formula (3-2). 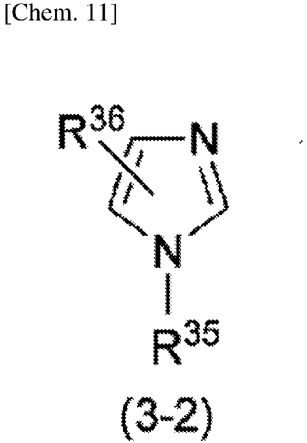 (in the formula, R³⁵ and R³⁶ each independently represent a hydrogen atom or an alkyl group)

Examples of alkyl groups for R³⁵ and R³⁶ include those exemplified as the alkyl groups for R³¹, R³², R³³ and R³⁴.

Preferable examples of imidazoles and imidazole derivatives include imidazoles and N-methylimidazole.

In addition, in addition to pyridine, pyridine derivatives, imidazole, and imidazole derivatives, preferable examples thereof include 1,4-diazabicyclo [2,2,2] octane (DABCO).

### <Process 3>

Process 3 is a process in which the cationically active species obtained in Process 2 is reacted with an amine to produce an amide.

In Process 3 in the method for producing an amide according to the embodiment, a cationically active species represented by the following General Formula (4) and an amine represented by the following General Formula (6) are reacted to obtain an amide represented by the following General Formula (7). 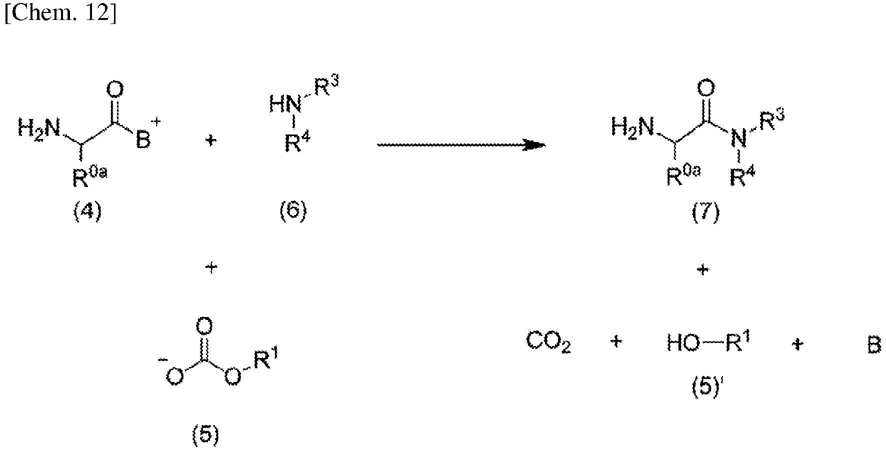 (R^{0a} in Formula (4) and Formula (7) has the same meaning as R^{0a} in Formula (2); R³ and R⁴ in Formula (6) and Formula (7) each independently represent a hydrogen atom or a monovalent organic group; and R¹ in Formula (5) has the same meaning as R¹ in Formula (2))

Here, in Process 2 and Process 3, alkoxide (O⁻-R¹) and CO₂ may be produced in place of Formula (5).

The amine is preferably an amino acid or an amino acid derivative.

Regarding the amino acid, the amino acid is preferably an α-amino acid. In addition, generally, since amino acids constituting peptides or proteins in a living body are of an L-type, the amino acids are preferably an L-type. The α-amino acid may be a compound represented by the following General Formula (6-1). 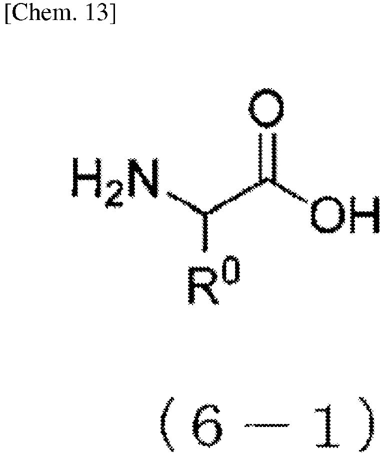 (in the formula, R⁰ represents a side chain of an amino acid)

The amino acids may be 20 types of amino acids which constitute peptides or proteins in a living body and are encoded as genetic information. These amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. In addition, the amino acid may be a type of amino acid that is not encoded as genetic information such as cysteine.

For example, R⁰ in Formula (1-1) is "-CH3" when the amino acid is alanine, "-H" when the amino acid is glycine, "-CH(CH₃)₂" when the amino acid is valine, and "-CH(CH₃)CH₂CH₃" when the amino acid is isoleucine. The same also applies to other amino acids.

When Formula (6) represents an amino acid, -R³ and -R⁴ may be, for example,-H and -CH(R⁰)COOH.

The amino acid need not be an α-amino acid. For example, it may be a β-amino acid such as β-alanine.

The amine may be an amino acid derivative. The amino acid derivative may be a compound having substantially the same properties as the amino acid, and may be a natural type that occurs naturally or a type which has modifications such as alternation, addition, or substitution of a functional group different from those of the natural type.

As an example of a case having substantially the same properties as an amino acid, a case in which amino acid derivatives can be incorporated into an enzyme that uses an amino acid as a substrate and a case in which amino acid derivatives can be bound to molecules that bind to an amino acid may be exemplified.

When the amine is arginine or an arginine derivative, two amino groups or imino groups in the side chain, which are shown as carboxylic acids in formation of the amide bonds above, are preferably arginines protected by protecting groups.

Examples of amino acid derivatives include those in which one or more hydrogen atoms or groups in the amino acid are substituted with other groups (substituents). As an example of amino acid derivatives, a protected amino acid in which a functional group is protected by a protecting group may be exemplified. Examples of sites protected by a protecting group include any one or more sites selected from the group consisting of amino groups, carboxylic groups, and side chains. One or two or more functional groups contained in the side chain may be protected by a protecting group. In Process 3, it is preferable that carboxylic groups and/or functional groups in the side chain be protected so that the reaction of the reactive functional group other than amino groups is prevented.

The type of the protecting group is not particularly limited, and can be appropriately selected depending on the type of the functional group to be protected. Carboxylic groups may be protected by simply being neutralized in the form of salts, but are generally protected in the form of esters. Examples of esters include benzyl esters (sometimes abbreviated as Bn or BZl) in addition to alkyl esters such as methyl and ethyl, and the present invention is not limited thereto.

In the method for producing an amide according to the embodiment, the cationically active species is reacted with an amine in Process 3. Here, the method for producing an amide according to the embodiment has an advantage that the reaction rate does not depend on the nucleophilicity of the amine due to high electrophilicity of the cationically active species.

Therefore, the method for producing an amide according to the embodiment is suitable for the reaction with an amine having low nucleophilicity. Specific examples of amines having low nucleophilicity may include amines having a nucleophilicity lower than those of 18 amino acids excluding valine and isoleucine from 20 amino acids which constitute proteins and are encoded as genetic information, and more specific examples thereof can include valine, isoleucine, an N-alkylated amino acid, and derivatives thereof. The N-alkylated amino acid may be one in which one or two hydrogen atoms of an amino group bonded to α carbon are substituted with an alkyl group and is preferably N-methyl amino acid in which one hydrogen atom is substituted with a methyl group. In the related art, these amines having low nucleophilicity are difficult to use for synthesis in an acid anhydride method. However, according to the method for producing an amide of the embodiment, it is possible to use amines having low nucleophilicity which have been difficult to use for synthesis in an acid anhydride method in the related art, and in this respect as well, the method for producing an amide according to the embodiment is revolutionary.

For example, a mixed acid anhydride method is performed under conditions shown in Example 1, the mixed acid anhydride produced in Example 1 is reacted with an amine whose nucleophilicity is desired to be determined, and the nucleophilicity of the amine here can be determined from the degree of reaction efficiency.

In the present embodiment, the amount of each compound used during the reactions in Processes 1 to 3 may be appropriately adjusted according to a desired reaction in consideration of the types of these compounds.

A molar equivalent ratio (carboxylic acid:amine) between the carboxylic acid and the amine in the reaction system may be 10:1 to 1/10:1, 5:1 to 1/5:1, or 3:1 to 1/3:1. According to the method for producing an amide of the embodiment, even if a relatively small amount of an amine, which is close to an equivalent amount, is reacted with the carboxylic acid, it is possible to produce an amide with high efficiency.

In the present embodiment, the reaction time for each process may be appropriately adjusted according to other conditions such as the reaction temperature. As an example, the reaction time in Process 1 may be 0.5 seconds to 30 minutes, 1 second to 5 minutes, or 3 seconds to 1 minute. When Process 2 and Process 3 are performed simultaneously, the reaction time for Process 2 and Process 3 may be 1 second to 60 minutes, 5 seconds to 30 minutes, or 1 minute to 10 minutes.

In the present embodiment, the temperature (reaction temperature) during the reactions in Processes 1 to 3 may be appropriately adjusted according to the type of compounds used in Processes 1 to 3. As an example, the reaction temperature is preferably in a range of 0 to 100 °C and more preferably in a range of 20 to 50 °C.

In the present embodiment, the reactions in Process 1 to Process 3 may be performed in the coexistence of a solvent. The solvent is not particularly limited, and a solvent that does not interfere with a reaction of a compound is preferable, and a solvent in which raw materials used in a reaction have high solubility is preferable. Examples thereof include N,N-dimethylformamide (DMF), tetrahydrofuran (THF), and 1,4-dioxane.

In the present embodiment, in the reactions in Process 1 to Process 3, the reaction system may further contain other compounds that do not correspond to the above exemplified compounds in a range in which amide production can be achieved.

In the present embodiment, the reactions in Process 1 to Process 3 may be performed separately or simultaneously. In order to more effectively minimize the production of side-reaction products, it is preferable that Process 2 and Process 3 be performed simultaneously.

In the method for producing an amide according to the embodiment described above, the presence and structure of the product can be confirmed by measuring the spectrum obtained by analysis through NMR, IR, mass spectrometry, or the like or elemental analysis or the like. In addition, as necessary, the product may be purified and can be produced by a purification method such as distillation, extraction, recrystallization, and column chromatography.

According to the method for producing an amide of the embodiment, it is possible to produce an amide with very high efficiency. Even the acid anhydride obtained in Process 1 is in a state in which it accepts a nucleophilic species (amine) as an active species. In this method, a cationically active species is additionally formed in Process 2, and the amine is reacted with this for the first time. Since the cationically active species produced here has significantly higher activity than the acid anhydride, the reaction can proceed at a very high rate. It is thought that, in a conventional method, since the activity of the cationically active species is high, when the side chain is protected by only one protecting group, a function of protecting the side chain is probably insufficient, and it is not possible to minimize the occurrence of side reactions. On the other hand, according to the method for producing an amide of the embodiment, when two amino groups or imino groups in the side chain of arginine used as a carboxylic acid are protected by protecting groups, it is possible to significantly minimize the production of byproducts as compared with a conventional method. In addition, even with an amine having low reactivity, which was difficult to react in the conventional method, it is possible to easily cause the reaction to proceed.

### [Second embodiment]

The method for producing an amide according to the embodiment includes reacting arginines in which two amino groups or imino groups in the side chain are protected by protecting groups with a halogenated formate ester and then reacting with an amine. The method for producing an amide according to the embodiment may be a method including mixing a product obtained by reacting a mixture obtained by mixing arginines and a halogenated formate ester and an amine.

The production method may include the following Process 1 and Process 3'. Process 1: a process in which arginines in which two amino groups or imino groups in the side chain are protected by a protecting group are reacted with a halogenated formate ester to obtain a mixed acid anhydride.
Process 3': a process in which the mixed acid anhydride obtained in Process 1 is reacted with an amine to produce an amide.

The above processes will be described below.

Parts the same as those in the first embodiment will not be described below.

### <Process 1>

Since Process 1 in the second embodiment is the same as Process 1 in the first embodiment, description thereof will be omitted.

### <Process 3'>

Process 3' is a process in which the mixed acid anhydride obtained in Process 1 is reacted with an amine to produce an amide.

In Process 3' in the method for producing an amide according to the embodiment, a mixed acid anhydride represented by the following General Formula (2) is reacted with an amine represented by the following General Formula (6) to obtain an amide represented by the following General Formula (7). 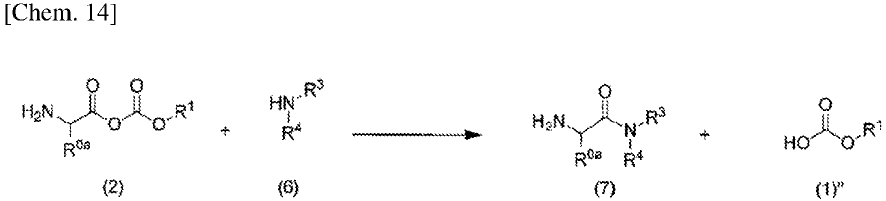 (in the formula, R^{0a} represents a side chain of arginines, R¹ represents a hydrocarbon group, and R³ and R⁴ each independently represent a hydrogen atom or a monovalent organic group)

In the method for producing an amide according to the first embodiment, a cationically active species is reacted with an amine to produce an amide. On the other hand, in the method for producing an amide according to the second embodiment, a mixed acid anhydride is reacted with an amine to produce an amide.

Regarding reaction conditions and the like in the method for producing an amide according to the second embodiment, the reactions in Process 1 to Process 3 described in the first embodiment can be read as Process 1 and Process 3'.

In the present embodiment, the reactions in Process 1 and Process 3' may be performed separately or simultaneously. In order to more effectively minimize the production of side-reaction products, it is preferable to simultaneously perform Process 1 and Process 3'.

When the two amino groups or imino groups are protected, side reactions including isomerization and δ-lactam production are significantly minimized in activation conditions via the acid anhydride.

According to the method for producing an amide of the present embodiment, it is possible to significantly minimize the production of byproducts and it is possible to produce an amide with high efficiency. It is thought that a reaction rate is faster in the method for producing an amide according to the first embodiment than in the method for producing an amide according to the second embodiment. However, in the method for producing an amide according to the second embodiment, since side reactions are effectively minimized, it is possible to produce an amide with much higher efficiency than in the conventional method (symmetric acid anhydride method).

### «Method for producing peptide»

In the method for producing an amide according to the embodiment, when the amine is an amino acid or an amino acid derivative, peptides or proteins can be synthesized. The method for producing an amide includes a method for producing peptides or proteins.

The amide obtained in Process 3 is used as a carboxylic acid in Process 1, after Processes 1 to 3, Processes 1 to 3 are additionally repeated, and a polypeptide chain can be extended.

That is, the carboxylic acid also includes a polypeptide and the arginines (carboxylic acid) according to the embodiment also include arginines (carboxylic acid) positioned at the C-terminal as a structural unit of the polypeptide. In this manner, the method for producing an amide according to the embodiment is suitable as a method for producing peptides or proteins.

### «Distribution system reaction device»

The method for producing an amide according to the embodiment can be performed using a distribution system reaction device. A distribution system reaction device including flow paths for transporting a fluid containing raw materials or an intermediate used in the reaction in the method for producing an amide according to the embodiment and a mixing machine for mixing the fluid may be exemplified.

Using the first embodiment as an example, regarding use of the distribution system reaction device, for example, a reaction with an amine in at least Process 3 may be performed in the distribution system reaction device, reactions of reacting with a base and reacting with an amine in Process 2 and Process 3 may be performed in the distribution system reaction device, and reactions in which, in Processes 1 to 3, arginines in which two amino groups or imino groups in the side chain are protected by protecting groups are reacted with a halogenated formate ester and then reacted with a base and reacted with an amine may be performed in the distribution system reaction device.

Using the second embodiment as an example, a reaction with an amine in at least Process 3' may be performed in the distribution system reaction device, and reactions in which, in Processes 1 and 3', arginines in which two amino groups or imino groups in the side chain are protected by protecting groups are reacted with a halogenated formate ester and then reacted with an amine may be performed in the distribution system reaction device.

Here, the method for producing an amide according to the embodiment is not limited to the method that is performed using the distribution system reaction device. For example, a batch container having a small volume and a high stirring speed may be used. The volume of the mixing part of the batch container may be 1 to 100 mL or 5 to 50 mL.

Hereinafter, a form of a distribution system reaction device according to an embodiment and a method for producing an amide according to a first embodiment using the same will be described with reference to Fig. 1.

Fig. 1 is a schematic view showing a schematic configuration of a distribution system reaction device 1. The distribution system reaction device 1 includes a tank 11 in which a first liquid is accommodated, a tank 12 in which a second liquid is accommodated, and a tank 13 in which a third liquid is accommodated.

As an example, the first liquid may contain arginines, the second liquid may contain a halogenated formate ester, and the third liquid may contain a base and an amine. As an example, the first liquid may contain a reagent which activates arginines and a halogenated formate ester, the second liquid may contain a halogenated formate ester, and the third liquid may contain a base and an amine. As a more specific example, as shown in Fig. 1, the first liquid contains arginines (carboxylic acid) in which two amino groups or imino groups in the side chain are protected by protecting groups, N-methylmorpholine, and DIEA, the second liquid contains isopropyl chloroformate, and the third liquid contains 4-morpholinopyridine and an amine.

As an example, in case of a method for producing an amide according to second embodiment, the third liquid may contain an amine.

If the first embodiment is used as an example, regarding use of the distribution system reaction device, for example, a mixture containing at least the first liquid and the second liquid may be mixed with the third liquid in the distribution system reaction device, and additionally, the first liquid and the second liquid may be mixed in the distribution system reaction device.

The distribution system reaction device 1 includes flow paths f1, f2, f3, f4, and f5 for transporting a fluid. As an example, the inner diameter of the flow path may be 0.1 to 10 mm or 0.3 to 8 mm. The distribution system reaction device 1 includes mixing machines 31 and 32 for mixing fluids. As an example, the inner diameter of the flow path inside the mixing machine may be 0.1 to 10 mm or 0.3 to 8 mm. Examples of mixing machines include a static mixer having no drive unit. A drive unit is a unit that receives power and moves.

The inner diameter of the flow path can be a diameter of the inner portion (a portion through which a fluid passes) of the flow path in the cross section of the flow path in a direction perpendicular to the length direction of the flow path. When the shape of the inner portion of the flow path is not a perfect circle, the inner diameter of the flow path can be a diameter when the shape of the inner portion of the flow path is converted into a perfect circle based on the area.

As an example, the tanks 11, 12, 13, and 14, the mixing machines 31 and 32, and the flow paths f1, f2, f3, f4, and f5 are formed of a resin such as a plastic or an elastomer or a glass material, a metal, a ceramic, or the like.

The tank 11 is connected to a pump 21, and the first liquid accommodated in the tank 11 moves through the flow path f1 due to an operation of the pump 21 and flows into the mixing machine 31. The tank 12 is connected to a pump 22, and the second liquid accommodated in the tank 12 moves through the flow path f2 due to an operation of the pump 22 and flows into the mixing machine 31. Then, the first liquid and the second liquid are mixed by the mixing machine 31 to form a first mixed liquid and the first mixed liquid is sent to the flow path f4. In a procedure after this mixing, carboxylic acid contained in the first liquid and isopropyl chloroformate contained in the second liquid are dehydrated and condensed to obtain a mixed acid anhydride (Process 1 in the method for producing an amide). The first mixed liquid containing the obtained acid anhydride flows into the mixing machine 32.

On the other hand, the tank 13 is connected to a pump 23, the liquid accommodated in the tank 13 moves through the flow path f3 due to an operation of the pump 23, flows into the mixing machine 32, and is mixed with the first mixed liquid to form a second mixed liquid, and the second mixed liquid is sent to the flow path f5. In a procedure after this mixing, the mixed acid anhydride obtained in Process 1 reacts with 4-morpholinopyridine contained in the third liquid to form a cationically active species (Process 2 in the method for producing an amide), and subsequently, the obtained cationically active species reacts with an amine contained in the third liquid to obtain an amide (Process 3 in the method for producing an amide). The second mixed liquid containing the produced amide is stored in a tank 14.

According to the distribution system reaction device 1 of the embodiment, it is possible to increase an area for performing heat exchange per volume of the reaction solution. In addition, it is possible to control the reaction time by the flow rate and the length of the flow path. Therefore, it is possible to precisely control the reaction solution, and as a result, it is possible to minimize the progress of undesired side reactions, and it is possible to improve the yield of the desired product.

Since the cationically active species obtained in Process 2 has high activity, it has an advantage that it can also be reacted with an amine having low reactivity, but it is important to control the reaction. In addition, since the mixed acid anhydride obtained in Process 1 has sufficiently high activity, it is important to control the reaction.

According to the distribution system reaction device 1 of the embodiment, when liquids are continuously distributed through the flow paths, an opportunity for compound collision is improved, the reaction can proceed with higher efficiency, and it is easy to minimize side reactions. For example, since the mixed acid anhydride produced in Process 1 can be immediately reacted with 4-morpholinopyridine (base), the time during which the mixed acid anhydride is in an activated state can be shortened, and it is possible to reduce a probability of the occurrence of side reactions such as isomerization.

Here, in the distribution system reaction device according to the present embodiment, the form in which liquids are mixed by a mixing machine has been exemplified. However, since liquids can be mixed simply by communicating the flow paths with each other, the distribution system reaction device of the embodiment does not necessarily include the mixing machine.

As shown here, the method for producing an amide according to the embodiment can be performed by a liquid phase method. For example, a current mainstream method for producing peptides (amides) is a solid phase method, and peptides in a solid phase may be synthesized. On the other hand, the liquid phase method is suitable for large-scale synthesis, and has favorable reactivity because the degree of freedom of molecules is high. The liquid phase method is also effective in reacting with an amine having low reactivity.

Here, in the distribution system reaction device according to the present embodiment, 5 types of compounds to be reacted are separately accommodated in three tanks. However, for example, the compounds may be accommodated in a total of 5 separate tanks and mixed sequentially.

However, as shown as the third liquid of the above embodiment, preferably, 4-morpholinopyridine (base) and an amine are present in the same liquid in advance. That is, Process 2 and Process 3 may be performed simultaneously, and accordingly, it is easy to react immediately the cationically active species having high reactivity produced in Process 2 with a desired amine, the time during which the cationically active species is in an activated state can be shortened, and it is possible to effectively minimize the production of undesired side-reaction products.

The method for producing an amide according to the second embodiment can be similarly performed by using the distribution system reaction device. In that case, it is preferable that the halogenated formate ester and the amine are existed in the same liquid in advance. That is, Process 1 and Process 3' may be performed at the same time. By doing this, it becomes easy to make the mixed acid anhydride produced in Process 1 immediately react with the desired amine. Then, it is possible to shorten the time during which the mixed acid anhydride is in an activated state, and it is possible to effectively minimize the production of side-reaction products.

While embodiments of the invention have been described above in detail with reference to chemical formulae and drawings, configurations and combinations thereof in the embodiments are only examples, and configurations can be added, omitted, and replaced and other modifications can be made without departing from the spirit and scope of the present invention. In addition, the present invention is not limited to the embodiments, but is limited only by the scope of claims.

### [Examples]

While the present invention will be described below in more detail with reference to examples, the present invention is not limited to the following examples.

### <Example 1> Mixed acid anhydride method/used of Fmoc-Arg(Cbz)₂-OH [raw materials]

Regarding the amino acid used as a carboxylic acid, Fmoc-Arg(Cbz)₂-OH (commercial product) which is arginine in which an amino group is protected by an Fmoc group and two side chains are protected by a Cbz group was used. Regarding the amino acid used as an amine, H-MePhe-OMe (commercial product) which is phenylalanine in which a carboxylic group is protected by a methyl group and the amino group is methylated was used.

### [Flow synthesis of acid amide]

A coupling reaction between the amino acid used as a carboxylic acid and the amino acid used as an amine was caused. For the coupling reaction, a distribution system reaction device composed of a PTFE tube (an inner diameter of 0.8 mm and an outer diameter of 1.59 mm) and a T-shaped mixer was used. Three unreacted solutions were separately prepared. The first solution was obtained by dissolving Fmoc-Arg(Cbz)₂-OH used as a carboxylic acid, N-methylmorpholine(NMM), and DIEA in 1,4-dioxane. The second solution was obtained by dissolving isopropyl chloroformate in 1,4-dioxane. The third solution was obtained by dissolving H-MePhe-Ome used as an amine and 4-morpholinopyridine in 1,4-dioxane. A molar equivalent ratio in the flow reaction system was 1.0 for H-MePhe-OMe, 0.010 for 4-morpholinopyridine and 1.0 for the remaining Fmoc-Arg(Cbz)₂-OH, N-methylmorpholine, DIEA, and isopropyl chloroformate.

In order to perform coupling in the flow system, first, the first solution and the second solution were mixed in a T-shaped mixer and reacted in the flow system for 5 seconds to obtain a mixed acid anhydride. Immediately thereafter, a reaction solution containing the mixed acid anhydride and the third solution were mixed using a new T-shaped mixer, and reacted in the flow system for 30 seconds and in a test tube for about 5 minutes after collection. All of these reactions were performed at 40 °C, and 20 seconds was set as a time for heat exchange before the unreacted solutions reached the mixer. Various solutions were discharged using a syringe pump, and the flow rate of each pump was 1.2 mL/min for the first solution, 2.0 mL/min for the second solution, and 2.0 mL/min for the third solution.

The reaction in Process 1 in the method for producing an amide in Example 1 is shown below. 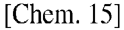 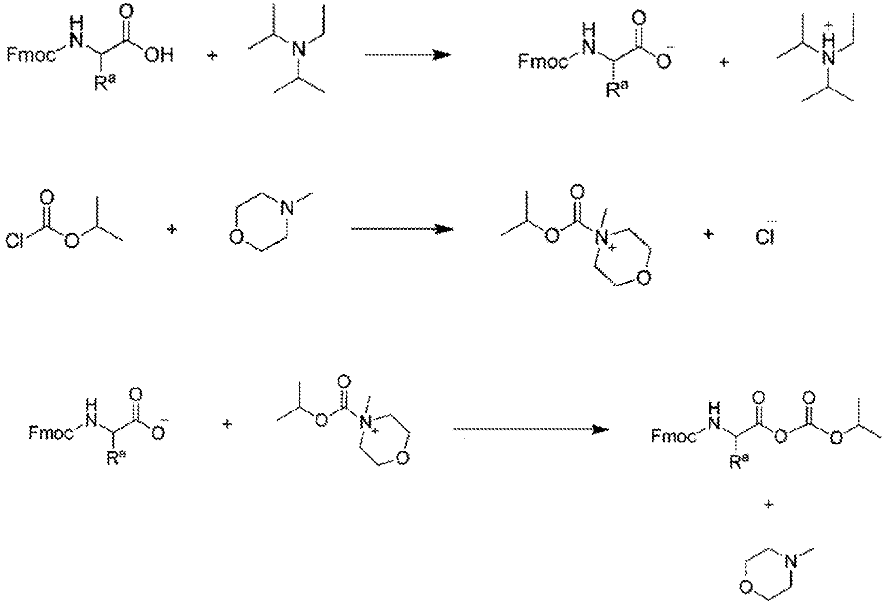 [in the formula, R^{a} represents an arginine side chain (in the present example, two groups corresponding to Z¹ and Z² among groups represented by General Formula (R^{0a-}b)are protected by the protecting group Cbz)]

The reaction in Process 2 in the method for producing an amide in Example 1 is shown below. 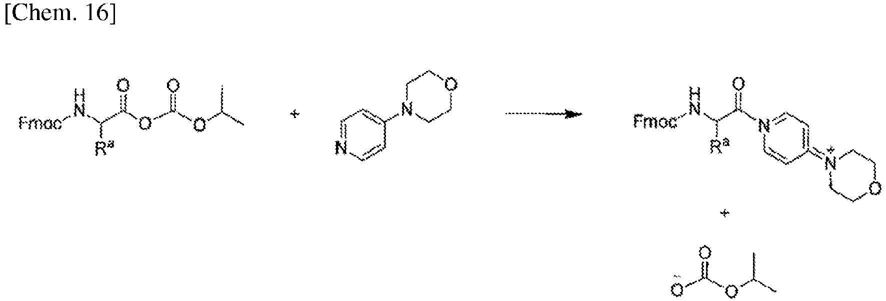 [in the formula, R^{a} represents an arginine side chain (in the present example, two groups corresponding to Z¹ and Z² among groups represented by General Formula (R^{0a-}b)are protected by the protecting group Cbz)]

The reaction in Process 3 in the method for producing an amide in Example 1 is shown below. 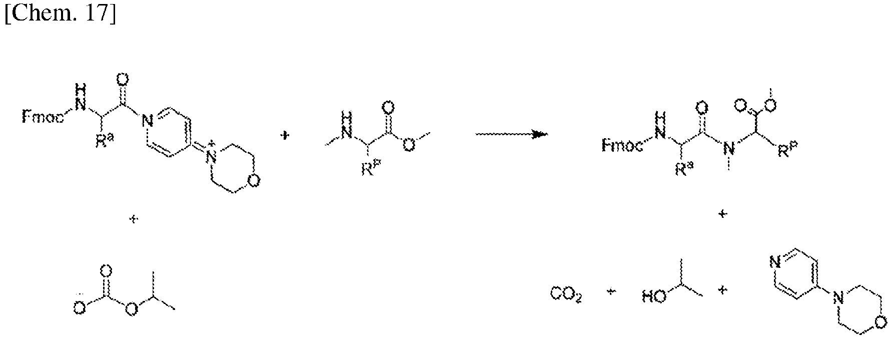 [in the formula, R^{a} represents an arginine side chain (in the present example, two groups corresponding to Z¹ and Z² among groups represented by General Formula (R^{0a-}b) are protected by the protecting group Cbz) and R^{p} represents a phenylalanine side chain]

### [Analysis method]

The desired product was isolated using column chromatography and identification was performed through H¹-NMR at 400 MHz.

Analysis of the isomerization rate was performed using GC-MS.

The sample was prepared as follows. After protecting groups of the obtained dipeptide were removed, the peptide/amino acid derivatives were hydrolyzed in deuterium hydrochloric acid, the sample was esterified with deuteride in methyl alcohol, a reagent was evaporated, and the residue was then acylated using trifluoroacetic anhydride or pentafluoropropionic anhydride.

The yield of the desired product was calculated from the weight of the isolated and purified desired product. That is, the molar equivalent ratio of the amine was set to 1.0, and a ratio of amine coupling was calculated from the weight of the isolated dipeptide.

### [Results]

NMR data of the obtained dipeptide is shown below.
¹H NMR (400 MHz, CDCl₃, major rotamer):δ 9.45 (brs, 1 H), 9.24 (brs, 1 H), 7.36-7.07 (m, 15 H), 5.21-5.11 (m, 6 H), 4.45-4.41 (m, 1 H), 3.98-3.96 (m, 2 H), 3.63 (s, 3 H), 3.37-3.32 (m, 1 H), 2.99-2.93 (m, 1 H), 2.79 (s, 3 H), 1.69-1.60 (m, 2 H), 1.45-1.39 (m, 10 H), 1.12-1.07 (m, 1 H).

Analyzing the product after the reaction showed that the dipeptide that was the desired product had a coupling yield of 85%, of which an isomerization ratio of the Arg site was 0.5%.

According to the method in Example 1, although the molar equivalent ratio of the carboxylic acid to the amine was 1:1, a high coupling yield of 80% or more was obtained in a short time of 5 minutes. In addition, the generation rate of the epimer contained in the desired product was 1% or less.

### <Comparative Example 1> Symmetric acid anhydride method/use of Boc-Arg(NO₂)-OH [raw material]

Regarding the amino acid used as a carboxylic acid, Boc-Arg(NO₂)-OH which is arginine in which the amino group is protected by the Boc group and the arginine side chain is protected by the NO₂ group was used. Regarding the amino acid used as an amine, H-MePhe-OMe which is phenylalanine in which the carboxylic group is protected by the methyl group and the amino group is methylated.

### [Flow synthesis of acid amide]

A coupling reaction between the amino acid used as a carboxylic acid and the amino acid used as an amine was caused. For the coupling reaction, a distribution system reaction device composed of a PTFE tube (an inner diameter of 0.8 mm and an outer diameter of 1.59 mm) and a T-shaped mixer was used. Three unreacted solutions were separately prepared. The first solution was obtained by dissolving Boc-Arg(NO₂)-OH used as a carboxylic acid and DIEA in DMF. The second solution was obtained by dissolving triphosgene in MeCN. The third solution was obtained by dissolving H-MePhe-OMe in MeCN. A ratio of molar concentrations in the distribution system reaction device was 1.0 for H-MePhe-OMe, 0.40 for triphosgene, 3.0 for DIEA, and 2.5 was for carboxylic acid.

In order to perform coupling in the distribution system reaction device, first, the first solution and the second solution were mixed in a T-shaped mixer and reacted in the distribution system reaction device for 1 second to obtain an acid anhydride.

Immediately thereafter, a reaction solution containing the acid anhydride and the third solution were mixed using a new T-shaped mixer, and reacted in the distribution system reaction device for 10 seconds and in a test tube for about 90 minutes after collection. All of these reactions were performed at 20 °C, and 20 seconds was set as a time for heat exchange before the unreacted solutions reached the mixer. Various solutions were discharged using a syringe pump, and the flow rate of each pump was 2.0 mL/min for the first solution, 1.2 mL/min for the second solution, and 2.0 mL/min for the third solution.

### [Analysis method and results]

In isolation of the desired product, the reaction solution was treated with an acid and a base, and isolation was then performed using an auto column (commercially available from Biotage) and identification was performed through H¹-NMR at 400 MHz.

Two major compounds were isolated and one major compound was dipeptide (Boc-Arg(NO₂)-MePhe-OMe) as a desired product. The coupling yield was 39%, of which an isomerization ratio of the Arg site was separated by a chiral column (HPLC) and the isomerization ratio was 14.1%. The second major compound was δ-lactam which was a byproduct obtained by a primary reaction from the acid anhydride state. Assuming that all triphosgenes were consumed, a ratio of produced δ-lactam determined based on the amount of acid anhydride produced was 46%.

As a result, carboxylic acids were activated to obtain an acid anhydride in order to perform coupling, but the coupling and side reaction proceeded competitively. Accordingly, it was confirmed that about 50% of the acid anhydrides were consumed in the side reaction, and ad a result, the coupling efficiency was 50% or less.

Here, there was a difference in which an amide was synthesized by the mixed acid anhydride method in Example 1 and an amide was synthesized by the symmetric acid anhydride method in Comparative Example 1. However, it was thought that, since the acylpyridinium species produced by the mixed acid anhydride method had higher activity than the symmetric acid anhydride produced by the symmetric acid anhydride method, even if the mixed acid anhydride method was used as in Example 1, when Boc-Arg(NO₂)-OH was used as the carboxylic acid, many byproducts were generated naturally.

### <Comparative Example 2> Symmetric acid anhydride method/ use of Boc-Arg(Cbz)₂-OH [raw material]

Regarding the amino acid used as a carboxylic acid, Boc-Arg(Cbz)₂-OH which is arginine in which the amino group was protected by the Boc group and two arginine side chains were protected by the Cbz group was used. Regarding the amino acid used as an amine, H-MePhe-OMe which is phenylalanine in which the carboxylic group is protected by the methyl group and the amino group was methylated was used.

### [Flow synthesis of acid amide]

A coupling reaction between the amino acid used as a carboxylic acid and the amino acid used as an amine was caused. For the coupling reaction, a distribution system reaction device composed of a PTFE tube (an inner diameter of 0.8 mm and an outer diameter of 1.59 mm) and a T-shaped mixer was used. Three unreacted solutions were separately prepared. The first solution was obtained by dissolving Boc-Arg(Cbz)₂-OH used as a carboxylic acid and DIEA in DMF. The second solution was obtained by dissolving triphosgene in MeCN. The third solution was obtained by dissolving H-MePhe-OMe used as an amine in MeCN. A molar equivalent ratio in the distribution system reaction device was 1.0 for H-MePhe-OMe, 0.40 for triphosgene, 3.0 for DIEA, and 2.5 for Boc-Arg(Cbz)₂-OH used as a carboxylic acid.

In order to perform coupling in the flow system, first, the first solution and the second solution were mixed in a T-shaped mixer and reacted in the distribution system reaction device for 1 second to obtain an acid anhydride. Immediately thereafter, a reaction solution containing the acid anhydride and the third solution were mixed using a new T-shaped mixer, and reacted in the distribution system reaction device for 10 seconds at 0 °C, and in a test tube for about 40 minutes after collection at room temperature (about 24 °C). In all of these reactions, 20 seconds was set as a time for heat exchange before the unreacted solutions reached the mixer. Various solutions were discharged using a syringe pump, and the flow rate of each pump was 2.0 mL/min for the first solution, 1.2 mL/min for the second solution, and 2.0 mL/min for the third solution.

### [Analysis method and results]

As a result of developing and analyzing the reaction solution by TLC, spots different from the raw material appeared and one product was confirmed. However, when water was added to the reaction solution, it was confirmed that the product decomposed and spots of the raw material became thick.

As a result, it was thought that, since the product was highly likely to be a symmetric acid anhydride, and protecting groups were introduced into two side chains of Arg and they produced a symmetric acid anhydride, due to bulkiness around the reaction point in the coupling reaction with the amine and in the δ-lactam production reaction, the reaction was stopped in the state of the symmetric acid anhydride which was generally an unstable intermediate.

Therefore, in the method of Comparative Example 2, when two protecting groups were introduced into the arginine side chain, it was possible to minimize the reaction of producing the byproduct δ-lactam, but the coupling reaction did not proceed and no amide was produced.

### [Reference Signs List]

1 ... Distribution system reaction device
11, 12, 13, 14 ... Tank
21, 22, 23 ... Pump
31, 32 ... Mixing machine
f1, f2, f3, f4, f5 ... Flow path

## Claims

1. A method for producing an amide, the method comprising: reacting arginines, arginine derivatives or arginine analogs in which two amino groups or imino groups in the side chain are protected by protecting groups with a halogenated formate ester and then reacting with an amine.

2. The method for producing an amide according to claim 1, the method comprising: reacting arginines, arginine derivatives or arginine analogs in which two amino groups or imino groups in the side chain are protected by protecting groups with a halogenated formate ester and then reacting with a base and reacting with an amine.

3. A method for producing an amide, the method comprising: mixing a product obtained by reacting a mixture obtained by mixing arginines, arginine derivatives or arginine analogs in which two amino groups or imino groups in the side chain are protected by protecting groups and a halogenated formate ester, and an amine.

4. The method for producing an amide according to claim 3, the method comprising: mixing a product obtained by reacting a mixture obtained by mixing arginines, arginine derivatives or arginine analogs in which two amino groups or imino groups in the side chain are protected by protecting groups and a halogenated formate ester, a base, and an amine.

5. The method for producing an amide according to claim 2 or 4, wherein the base is any one or more selected from the group consisting of pyridine, pyridine derivatives, imidazole, imidazole derivatives and 1,4-diazabicyclo [2,2,2] octane.

6. The method for producing an amide according to claim 2 or 4, wherein the base is any one or more selected from the group consisting of 4-morpholinopyridine, N,N-dimethyl-4-aminopyridine, 4-pyrrolidinopyridine, pyridine, 4-methoxypyridine, imidazole, N-methylimidazole and 1,4-diazabicyclo [2,2,2] octane.

7. The method for producing an amide according to any one of claims 1 to 6, wherein the two protecting groups are carbamate-based protecting groups or sulfonamide-based protecting groups.

8. The method for producing an amide according to any one of claims 1 to 7, wherein the halogenated formate ester is any one or more selected from the group consisting of isopropyl chloroformate, isobutyl chloroformate, isopropyl bromomate and isobutyl bromomate.

9. The method for producing an amide according to any one of claims 1 to 8, wherein the amine is an amino acid or an amino acid derivative.

10. The method for producing an amide according to any one of claims 1 to 9, wherein the nucleophilicity of the amine is lower than the nucleophilicity of 18 amino acids excluding valine and isoleucine from 20 amino acids which constitute proteins and are encoded as genetic information.

11. The method for producing an amide according to claim 9 or 10, wherein the amine is valine, isoleucine an N-alkylated amino acid, or derivatives thereof.

12. The method for producing an amide according to any one of claims 1 to 11, wherein the reaction with the amine is performed in a distribution system reaction device.

13. The method for producing an amide according to claim 12, wherein arginines, arginine derivatives or arginine analogs in which two amino groups or imino groups in the side chain are protected by protecting groups are additionally reacted with a halogenated formate ester in the distribution system reaction device.
